Europäisches Patentamt

European Patent Office

Office européen des brevets

(11) Veröffentlichungsnummer: **0 508 344 A2**

# (12) EUROPÄISCHE PATENTANMELDUNG

(21) Anmeldenummer: **92105899.6**

(22) Anmeldetag: **06.04.92**

(51) Int. Cl.5: **C12C 11/04**, C12G 3/08, C12M 1/40

(30) Priorität: **12.04.91 DE 4111879**

(43) Veröffentlichungstag der Anmeldung:
**14.10.92 Patentblatt 92/42**

(84) Benannte Vertragsstaaten:
**BE DE DK FR GB NL**

(71) Anmelder: **FORSCHUNGSZENTRUM JÜLICH GMBH**
**Wilhelm-Johnen-Strasse**
**W-5170 Jülich(DE)**

(84) **BE DE DK FR NL**

(71) Anmelder: **Schott Glaswerke**
**Hattenbergstrasse 10**
**W-6500 Mainz 1(DE)**

(84) **GB**

(72) Erfinder: **Aivasidis, Alexander, Dr.**
**Morschenicher Strasse 37a**
**W-5170 Jülich(DE)**
Erfinder: **Wandrey, Christian, Prof.**
**Wolfshovener Strasse 139**
**W-5170 Jülich(DE)**
Erfinder: **Breitenbücher, Klaus, Dr.**
**Sunsweiler Strasse 22**
**W-6506 Nackenheim(DE)**
Erfinder: **Mistler, Manfred**
**Frauenlobstrasse 29 A**
**W-6500 Mainz(DE)**

(54) Verfahren zur Herstellung von alkoholfreiem Bier und dafür geeignete Vorrichtung.

(57) Alkoholfreies Bier wird durch kontinuierliche Fermentation im Wirbelschichtreaktor mit auf offenporigem Sinterglasträger von ≦ 5 mm Korngröße mit Poren zwischen 50 und 500 μm aufgewachsener Hefe bei Temperaturen zwischen 0 und 20°C bei niedriger Temperatur mit entsprechend der gewünschten geringen Alkoholkonzentration kontrollierter Verweilzeit erhalten. Bevorzugt werden Sinterglasträger mit Poren zwischen 50 und 300 insbesondere 60 und 250 μm und Porositäten zwischen 30 und 65 %. Korngrößen zwischen 0,5 - 4 mm insbesondere 1 - 3 mm werden bevorzugt. Die Fermentation findet insbesondere bei 0,5-10°C statt. Verweilzeiten zwischen 1 - 12 Std. insbesondere 2 - 8 Std. sind zweckmäßig. Die auf dem Träger aufgewachsenen Hefezellen werden insbesondere in einer Anfahrphase im Wirbelschichtreaktor durch Inokulation mit Hefekonzentrat bei 20 - 35°C insbesondere um 30°C und Verweilzeiten unterhalb der Generationszeit der Hefe über einige Tage hinweg angezüchtet. Das Schüttvolumen des Kornmaterials im Wirbelschichtreaktor liegt insbesondere bei 25 - 30 % des Arbeitsvolumens und das Wirbelschichtvolumen bei 40 - 60 % des Arbeitsvolumens. Überdrucke von 0,5 - 3 bar werden bevorzugt.

FIG. 1

Die Erfindung bezieht sich auf ein Verfahren zur Herstellung von Bier mit einem Alkoholgehalt ≦ 0,5 Vol% durch kontinuierliche Fermentation mit immobilisierter Hefe bei niedriger Temperatur mit entsprechend der geringen Alkoholkonzentration kontrollierter Verweilzeit, und sie umfaßt einen dafür geeigneten Reaktor.

Bier gilt als alkoholfrei, wenn der Ethanolgehalt weniger als 0,5 Vol% beträgt. Dies kann entweder durch einen nachträglichen Entzug von Ethanol aus einem weitgehend vergorenen Bier durch Einsatz von Dialysemembranen erzielt werden oder indem von vornherein durch kontrollierte Fermentationsbedingungen gerade nur so viel Alkohol gebildet wird. Diese letztere Methode stellt auch aufgrund der zunehmenden Verbrauchernachfrage nach alkoholfreiem Bier die Methode der Wahl dar. Die Gärungsaktivität der Hefe wird dabei über Temperatur und Verweilzeit soweit reguliert, daß der in der Würze vorhandene Zucker nur zu einem geringen Prozentsatz zu Ethanol umgesetzt wird. Typische Fermentationsbedingungen sind dabei eine Temperatur von 1,5°C und eine Verweilzeit von 17 h unter Batch-Bedingungen im Gärungstank.

Von H. Lommi (Brewing & Distilling Internat. 1990, 22-23) wurde auch bereits ein Verfahren zur Gewinnung von alkoholfreiem Bier beschrieben, bei dem auf einem heterogenen Träger aus DEAE-Cellulose, Polystyrol und Titandioxid aufgewachsene Hefe im Festbett kontinuierlich durchströmt wird.

Diese Verfahrensweise geht im Prinzip auf Entwicklungen zur kontinuierlichen Biergewinnung unter Verwendung von immobilisierten Hefezellen zurück, die seit dem Ende der 50er Jahre intensiv untersucht wird, aber noch in letzter Zeit aufgrund der bestehenden Erfahrungen als nicht restlos befriedigend kritisiert wird (A.W. Davies, Proc. Conv. Inst. Brew., Brisbane 1988 (20) 159-65).

Als Trägermaterialien werden neben Holzspähnen, PVC-Chips und Kieselgur im wesentlichen Alginatkügelchen oder DEAE-Cellulose-Körner für die Immobilisierung der Hefe verwendet. J.I. Centenera u.a. (Proc. Eur. Brew. Conv. Zürich 1989, 307-14) beschreiben auch die Verwendung von Polygalacturonsäure als Trägermaterial für aufgewachsene Hefe zur kontinuierlichen Biergewinnung.

Zahlreiche Arbeiten befassen sich im übrigen mit der Bildung von Begleitstoffen und geschmacklichen Einflüssen der Herstellungsbedingungen.

Trotz der lebhaften Entwicklungstätigkeit über die Jahre hinweg konnte bislang kein überzeugendes Verfahren entwickelt werden. Ziel der Erfindung ist daher ein Verfahren, mit dem alkoholfreies Bier kontinuierlich erzeugt werden kann und das über lange Zeiten hinweg zu einer Produktion gleichbleibender Güte führt.

Das zu diesem Zweck entwickelte erfindungsgemäße Verfahren ist dadurch gekennzeichnet, daß man die Fermentation im Wirbelschichtreaktor mit auf offenporigem Sinterglasträger von ≦ 5 mm Korngröße mit Poren zwischen 50 und 500 $\mu$m aufgewachsener Hefe bei Temperaturen zwischen 0 und 20°C durchführt.

Vorzugsweise werden dabei poröse Sinterglasträger von 0,5 - 4 mm, insb. 1 - 3 mm Korngröße und Porositäten im Bereich von 30 - 65 % verwendet. Die Porengrößen liegen zweckmäßigerweise zwischen 50 und 300 $\mu$m, insb. zwischen 60 und 250 $\mu$m.

Bevorzugte Temperaturen für die Fermentation liegen zwischen 1 und 15°C, insb. zwischen 4 und 10°C. Die Verweilzeiten richten sich nach dem gewünschten Gärungsresultat und liegen üblicherweise im Bereich von 1 - 12 Std. Besonders zweckmäßig sind Verweilzeiten von 2 - 8 Std.

Grundsätzlich sollten die Sinterglasträger möglichst große Poren und eine hohe Porosität aufweisen sowie eine relativ geringe Korngröße, die aus mechanischen Gründen umso größer sein muß, je größer die Poren sind. Zweckmäßig sind offenporige Sinterglasgranulate von 1 - 2 mm Korngröße, einer Porosität von ≧ 45 % und Poren von 80 - 250 $\mu$m.

Zwar wurde poröses Glas bereits 1977 (Navarro et al., European J. Appl. Microbiol. 4 - (1977) 243-54) zur Immobilisierung von Hefe in Betracht gezogen (desgl. DE-OS 28 39 580) und auch von der Anmelderin werden in der DE-OS 34 10 650 sowie in der DE-PS 36 39 153 poröse Gläser als Träger für Mikroorganismen beschrieben und auch die Möglichkeit zur Erzeugung von Gärungsprodukten sowie Anwendung im Festbettoder Wirbelschichtreaktor erwähnt, jedoch hat dieser globale Hinweis offensichtlich nicht richtungsweisend auf die Entwicklung spezieller Biergewinnungsverfahren gewirkt, für die bislang - insb. im Rahmen der Bierreifung - Alginate und Carrageenate sowie Cellulosederivate vorgesehen werden.

Als Reaktor eignet sich ein Wirbelschichtreaktor mit einer äußeren Umlaufpumpe, die für einen ausreichenden Durchfluß sorgt. Am oberen Ende hat der säulenförmige Reaktor eine Querschnittserweiterung, die für eine ausreichende Verlangsamung der Flüssigkeitsströmung und damit Umkehr des suspendierten Kornmaterials sorgt.

Bei einem solchen Reaktor macht das Wirbelschichtvolumen etwa 40 - 60 % des gesamten Arbeitsvolumens aus, und das Schüttvolumen liegt bei etwa 25 - 30 % dieses Arbeitsvolumens.

Mit einem solchen Wirbelschichtreaktor ist eine befriedigende Steuerung für eine gleichbleibende Qualität des Reaktorauslaufs gewährleistbar.

Die Mikroorganismen werden auf dem Trägermaterial vorzugsweise im Wirbelschichtreaktor in einer Anfahrphase angezüchtet, indem Hefekonzentrat bei 20 - 35°C, vorzugsweise bei ~30°C, als Inokulat in den Reaktor gegeben und mit Verweilzeiten unterhalb der Generationszeit der Hefe dafür gesorgt wird, daß während der Kolonisierungsphase des Trägermaterials ein optimaler Selektionsdruck zugunsten sessiler Mikroorganismen bei einem hohen Level der Raumzeitausbeute stattfinden kann. Zweckmäßigerweise erfolgt eine solche Anfahrphase über eine Woche hinweg und kann durch regelmäßige Probennahme und rasterelektronenmikroskopische Auswertung überwacht werden. Selbstverständlich sind je nach Bedingungen kürzere Anfahrphase von 2 - 3 Tagen bzw. auch längere Zeitdauern von bis zu mehreren Wochen möglich.

Beim erfindungsgemäßen Fermentationsprozeß wird der Alkoholgehalt über die Parameter Temperatur und Verweilzeit gesteuert, wobei Werte bis herab zu 0 - 1°C und 1 h anwendbar sind. Unter derartigen Bedingungen haben potentielle Kontaminanten auf Grund ihrer geringen Wachstumsgeschwindigkeit keine Chance, sich zu teilen und werden durch die hohe Durchflußrate ausgewaschen.

Das benutzte inerte Trägermaterial (insb. Borosilikat- oder Kalk-Natron-Glas) ist weitgehend biokompatibel, so daß die Verfahrensweise dem Reinheitsgebot beim Bierbrauen entspricht.

Das gewählte Trägermaterial von hoher offener Porosität und relativ geringer Teilchengröße ermöglicht die Zurückhaltung hoher Biomassekonzentrationen, welche um ein Mehrfaches über den beim trägerfreien konventionellen Verfahren mit Batch-Betrieb liegen. Die gemessenen Biotrockenmassegehalte liegen bei 25 - 30 g/l Wirbelschichtvolumen.

Zweckmäßigerweise wird mit Überdrucken von 0,5 bis 3 bar, insb. etwa 1,5 bar gearbeitet.

Nachfolgend wird die Erfindung anhand eines Ausführungsbeispiels näher beschrieben:

Die kontinuierliche Fermentation alkoholfreien Bieres wurde in zwei autoklavierbaren Labor-Wirbelschichtreaktoren mit einem Gesamtreaktionsvolumen von 2,0 l und einem Wirbelbettvolumen von ca. 0,9 l durchgeführt.

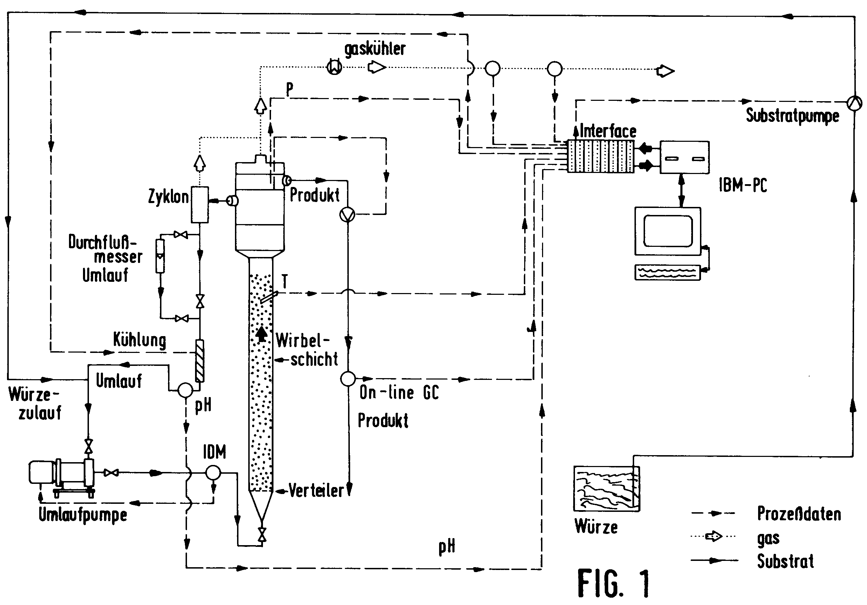

Als Trägermaterial diente Siran® -Granulat der Fa. Schott mit einer Teilchengrößenverteilung von 1 - 2 mm, einer Porosität von 55 % und einer Porengrößenverteilung von 30 - 120 $\mu$m bzw. 60 - 300 $\mu$m. Der experimentelle Aufbau entsprach im wesentlichen der beigefügten Figur 1.

Figur 1 zeigt einen Wirbelschichtreaktor in Produktion, dessen Zulauf aus einem Würze-Vorlagetank mittels einer Pumpe kontinuierlich entnommen wird. Die Umlaufpumpe sorgt für die Träger-Fluidisierung im Reaktor durch Einstellung einer Linergeschwindigkeit von 15 - 50 m/h. Die rezirkulierte Flüssigkeit passiert einen Wärmeaustauscher zur Einstellung der gewünschten Temperatur. Zur Konstanthaltung derselben ist der Wirbelschichtreaktor mit einer äußeren Wärmedämmung versehen.

Mit Hilfe einer konduktometrischen Niveauregelung wird eine Produktpumpe angesteuert, so daß der Reaktor bei konstantem Volumen betrieben wird.

Gestrichelt angedeutet ist eine rechnergestützte Prozeßsteuerung und -überwachung mit Hilfe eines Personal-Computers zur Variation der Verweilzeit um einen gewünschten Alkoholgehalt nach On line Konzentrationserfassung einzustellen.

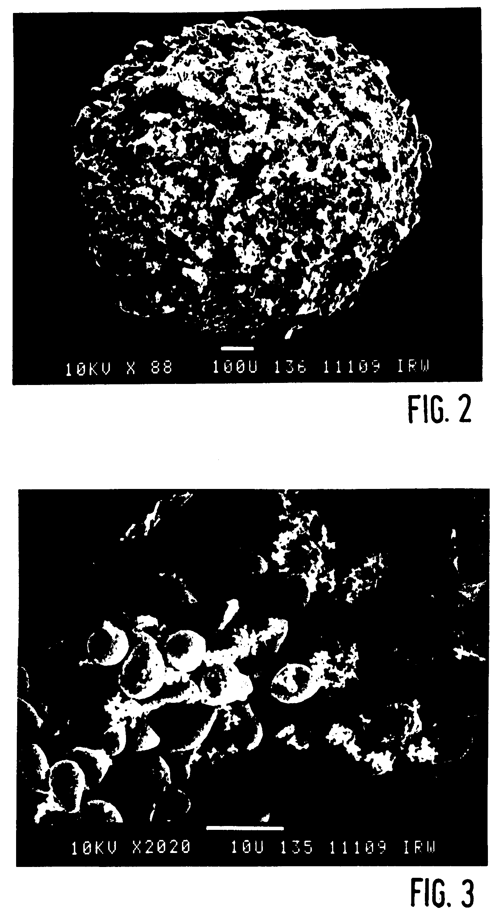

Figur 2 zeigt eine Siran® -Kugel nach einem vierwöchigen Reaktoreinsatz. Man erkennt die homogene Verteilung der Hefe in den durchgehenden großen offenen Poren.

Figur 3 zeigt eine dem gegenüber vergrößerte Aufnahme aus dem Kugelinnenraum. Man erkennt einen durchgehenden Bewuchs mit ausreichenden Lücken für einen guten Stofftransport zu den Mikroorganismen.

Die erforderliche Reinzuchthefe und Substrat stammten von der Brauerei Beck & Co.

Ergebnisse:

Die beiden Wirbelschichtreaktoren wurden nach Inokulation mit einem Hefekonzentrat in Betrieb genommen. Der Anfahrvorgang erfolgte anschließend bei einer Temperatur von 30°C eine Woche lang mit einer Verweilzeit von 4 Std.

Durch eine Reihe von vororientierenden Versuchen wurden anschließend die Betriebsbedingungen hinsichtlich Temperatur und Verweilzeit so weit eingegrenzt, daß sich im Reaktorablauf Alkoholkonzentrationen kleiner als der genannte Grenzwert für alkoholfreies Bier einstellten. Dabei resultierten Verweilzeiten von 2 - 5 h bei einer Temperatur von 1,5 - 4°C. Der Ethanolgehalt ist allerdings nur die eine meßtechnisch erfaßbare Leitgröße für die verordnungsmäßige Kennzeichnung eines alkoholfreien Bieres. Darüber hinaus wurden im stationären Zustand Proben in mikrofiltrierter Form entnommen und nach Aufkarbonisierung verkostet, die sich als geschmacklich weitgehend identisch mit dem herkömmlichen fermentierten alkoholfreien Bier erwiesen.

Die beiden Wirbelschichtreaktoren wurden bereits über 9 Monate lang störungsfrei betrieben. In diesem Zeitraum war kein nennenswerter Abrieb von Trägermaterial festzustellen. Eine Kontamination konnte nicht beobachtet werden, obwohl zeitweilig auch durch eine Wildhefe kontaminierte Würzechargen behandelt wurden. Die Siran®-Kugeln waren weitgehend mit Biomasse bewachsen. Ein Außenbewuchs der Kugeln war kaum feststellbar.

Seit Beginn der Versuche wurden mehr als 2 $m^3$ alkoholfreies Bier produziert. Die bisherigen reaktionstechnischen Untersuchungen haben gezeigt, daß eine kontinuierliche Biergärung mit an Siran® immobilisierter Hefe unter Verwendung von Wirbelschichtreaktoren möglich ist.

**Patentansprüche**

1. Verfahren zur Herstellung von Bier mit einem Alkoholgehalt ≤ 0,5 Vol% durch kontinuierliche Fermentation mit immobilisierter Hefe bei niedriger Temperatur mit entsprechend der geringen Alkoholkonzentration kontrollierter Verweilzeit,
   **dadurch gekennzeichnet,**
   daß man die Fermentation im Wirbelschichtreaktor mit auf offenporigem Sinterglasträger von ≤ 5 mm Korngröße mit Poren zwischen 50 und 500 $\mu$m aufgewachsener Hefe bei Temperaturen zwischen 0 und 20°C durchführt.

2. Verfahren nach Anspruch 1,
   **dadurch gekennzeichnet,**
   daß man Sinterglasträger mit Poren zwischen 50 und 300 $\mu$m und einer Porosität zwischen 30 und 65 % verwendet.

3. Verfahren nach Anspruch 2,
   **dadurch gekennzeichnet,**
   daß man Sinterglasträger mit Poren zwischen 60 und 250 $\mu$m verwendet.

4. Verfahren nach einem der vorangehenden Ansprüche,
   **dadurch gekennzeichnet,**
   daß man Sinterglasträger von 0,5 bis 4 mm, insb. 1 bis 3 mm Korngröße verwendet.

5. Verfahren nach einem der vorangehenden Ansprüche,
   **dadurch gekennzeichnet,**
   daß man die Fermentation bei 0,5 bis 10°C durchführt.

6. Verfahren nach einem der vorangehenden Ansprüche,
   **dadurch gekennzeichnet,**
   daß man mit Verweilzeiten von 1 bis 12 Std., insb. 2 - 8 Std. arbeitet.

7. Verfahren nach einem der vorangehenden Ansprüche,
   **dadurch gekennzeichnet,**
   daß man die auf dem Träger aufgewachsenen Hefezellen im Wirbelschichtreaktor in einer Anfahrphase durch Inokulation mit Hefekonzentrat bei 20 - 35°C und Verweilzeiten unterhalb der Generationszeit der Hefe über einige Tage hinweg auf dem Träger anzüchtet.

8. Verfahren nach Anspruch 7,
   **dadurch gekennzeichnet,**
   daß man das Anzüchten bei ~30°C über eine Woche hinweg durchführt.

9. Verfahren nach einem der vorangehenden Ansprüche,
   **dadurch gekennzeichnet,**
   daß man mit einem Schüttvolumen des Kornmaterials von 25 - 30 % des Arbeitsvolumens des Wirbelschichtreaktors arbeitet.

10. Verfahren nach Anspruch 9,
    **dadurch gekennzeichnet,**
    daß man mit einem Wirbelschichtvolumen von 40 - 60 % des Arbeitsvolumens arbeitet.

11. Verfahren nach einem der vorangehenden Ansprüche,
    **dadurch gekennzeichnet,**
    daß mit Überdrucken von 0,5 - 3 bar gearbeitet wird.

12. Wirbelschichtreaktor zur Durchführung des Verfahrens nach einem der vorangehenden Ansprüche,
    **gekennzeichnet durch**
    einen zu 25 - 30 % mit offenporigem Sinterglasträger von ≤ 5 mm Korngröße gefüllten Säulenreaktor mit oberer Querschnittserweiterung auf das etwa Doppelte und einer äußeren Umwälzpumpe für einen Durchfluß im flüssigkeitsgefüllten Reaktor zur Fluidisierung des enthaltenen Kornmaterials.

gaskühler

P

Interface

Substratpumpe

IBM-PC

Zyklon

Produkt

Durchflußmesser
Umlauf

T

Kühlung

Wirbelschicht

Umlauf

On-line GC

Würzezulauf

pH

Produkt

IDM

Verteiler

Umlaufpumpe

Würze

pH

Prozeßdaten

gas

Substrat

# FIG. 1

10KV X 88     100U 136 11109 IRW

**FIG. 2**

10KV X2020     10U 135 11109 IRW

**FIG. 3**